# EUROPÄISCHE PATENTANMELDUNG

(11) **EP 2 380 880 A2**
(43) Veröffentlichungstag der Anmeldung: **26.10.2011**
(21) Anmeldenummer: 10174320.1
(22) Anmeldetag: 27.08.2010
(51) Int. Cl.: C07D 233/58, C08K 5/19

(54) **Verfahren zur Herstellung konzentrierter Lösungen von Metallsalzen in ionischen Flüssigkeiten**

(30) Priorität: 01.09.2009 EP 09169111
(71) Anmelder: BASF SE, 67056 Ludwigshafen (DE)
(72) Erfinder: Stock, Christoph, 67158, Ellerstadt (DE); Tschirschwitz, Steffen, 68199, Mannheim (DE); Gerhard, Dirk, 68163, Mannheim (DE); Szarvas, Laszlo, 67071, Ludwigshafen (DE)

(57) **Zusammenfassung**

Verfahren zur Herstellung einer Lösung eines Metallsalzes in einer ionischen Flüssigkeit, **dadurch gekennzeichnet, dass**
- die ionische Flüssigkeit durch Alkylierung einer Ausgangsverbindung, welche mindestens ein Stickstoffatom enthält, hergestellt wird und die Alkylierung in Gegenwart des Metallsalzes erfolgt.

## Beschreibung

Die vorliegende Erfindung betrifft ein Verfahren zur Herstellung einer Lösung eines Metallsalzes in einer ionischen Flüssigkeit, welches **dadurch gekennzeichnet, dass** die ionische Flüssigkeit durch Alkylierung einer Ausgangsverbindung, welche mindestens ein Stickstoffatom enthält, hergestellt wird und die Alkylierung in Gegenwart des Metallsalzes erfolgt.

Für viele technische Verwendungen sind Mischungen von ionischen Flüssigkeiten und Metallsalzen von Bedeutung.

Derartige finden z.B. als Antistatika in Kunstoff-formteilen, Beschichtungsmitteln, z B. Lacken oder Bodenbeschichtungen, oder Klebstoffen Verwendung, wie z.B. DE-A 102006031952, DE-A 102006045869 und DE-A 102006015775 beschrieben ist. Insbesondere von Interesse sind dabei Mischungen von Imidazolium-alkylsulfaten mit Metallsalzen wie Natruimdicyanamid, Natriumtetrafluoroborat, Natriumhexafluorophosphat, Natriumthiocyanat oder den entsprechenden Kaliumverbindungen. Ein möglichst hoher Metallsalzgehalt in den Mischungen ist von Vorteil, weil so eine hohe Leitfähigkeit und damit eine sehr gute antistatische Wirkung erreicht wird.

Die Metallsalze sind nicht im gewünschten Maße in der ionischen Flüssigkeit löslich, so lösen sich z. B. nur ca. 8 Gew. teile Natriumdicyanamid (NaDCA) und nur 9 Gew. teile NaBF4 bei 25°C in Ethylmethylimidazolium-ethylsulfat (EMIM Ethylsulfat). Für Anwendungen als Antistatika sind aber häufig Lösungen mit mindestens 10 Gew. Teilen Metallsalz in der ionischen Flüssigkeit gewünscht oder notwendig, um eine ausreichend hohe Leitfähigkeit zu erreichen.

Eine Verwendung von heterogenen Mischungen, z. B. Suspensionen des Metallsalzes in der ionischen Flüssigkeit kommt im Allgemeinen nicht in Betracht, da derartige Mischungen nicht die erforderlichen anwendungstechnischen Eigenschaften haben.

Aus DE-A 102006031952 ist bekannt, Lösungsvermittler, beispielsweise Ethylenglycol oder andere Diole, zu verwenden, um höhere Gehalte an Metallsalzen einzustellen. Die Mischungen aus Metallsalz und ionischer Flüssigkeit enthalten dann aber eine zusätzliche Komponente, die gegebenenfalls vor oder während der späteren Verwendung wieder entfernt werden muss.

Aufgabe der vorliegenden Erfindung war ein einfaches Verfahren zur Herstellung von homogenen Mischungen aus ionischer Flüssigkeit und Metallsalz mit möglichst hoher Konzentration des Metallsalzes und guten anwendungstechnischen Eigenschaften.

Demgemäß wurde das oben definierte Verfahren gefunden.

Bei dem erfindungsgemäßen Verfahren werden ionische Flüssigkeiten durch eine Alkylierung von Ausgangsverbindungen hergestellt.

Als ionische Flüssigkeiten werden Salze bezeichnet, welche aus mindestens einem Kation und mindestens einem Anion bestehen und einen Schmelzpunkt kleiner 100°C (bei Normaldruck, d.h. 1 bar) haben; ionische Flüssigkeiten sind insbesondere Salze, welche bei 20°C (Normaldruck, 1 bar) flüssig sind.

Bekannte Ausgangsverbindungen, aus denen durch Alkylierung ionische Flüssigkeiten hergestellt werden können, sind insbesondere tertiäre Amine mit drei organischen Substituenten oder Verbindungen mit einem heterocyclischen Ringsystem, welches 1 bis 3 Stickstoffatome enthält.

Als derartige Ausgangsverbindungen bevorzugt sind:
ein tertiäres Amin der Formel I 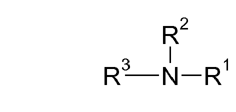
ein Imidazol der Formel II 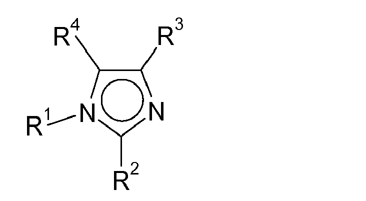 ein Pyridin der allgemeinen Formel III 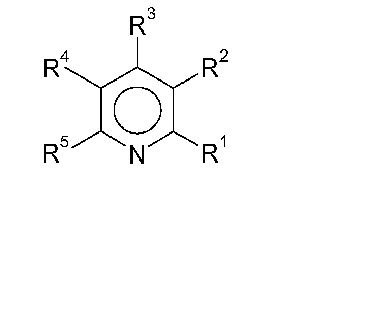 oder ein Pyrazol der allgemeinen Formel IV

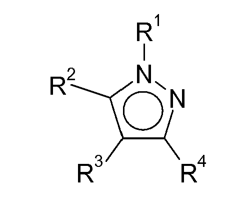

In den vorstehenden Formeln I bis IV stehen die Reste R1 bis R5 unabhängig voneinander für ein Wasserstoffatom oder eine organische Gruppe mit 1 bis 20 C-Atomen, insbesondere mit 1 bis 10 C-Atomen. Besonders bevorzugt stehen R1 bis R5 unabhängig voneinander für ein Wasserstoffatom oder einen Kohlenwasserstoffrest mit 1 bis 20 C-Atomen, insbesondere mit 1 bis 10 C-Atomen.

Im Falle des tertiären Amins der Formel I stechen jedoch mindestens zwei der Reste R1 bis R3 nicht für ein H-Atom.

Bevorzugte Imidazole der Formel II sind solche, in denen R1 für eine C1 bis C10 Alkylgruppe steht und R2, R3, R4 unabhängig voneinander für ein H-Atom oder eine C1 bis C10 Alkylgruppe stehen, wobei vorzugsweise mindestens zwei der Reste R2, R3 und R4 für ein H-atom stehen.

Ganz besonders bevorzugte Imidazole der Formel II sind solche, in denen R1 für eine C1 bis C4 Alkylgruppe und R2, R3 und R4 jeweils für ein H-Atom stehen.

Genannt sei z.B. 1-Methylimidazol und insbesondere 1-Ethylimidazol (R1=Ethyl, R2=H, R3=H, R4=H).

Bevorzugte Pyridine der Formel III sind solche, in denen R1 bis R5 unabhängig voneinander für ein H-Atom oder eine C1 bis C10 Alkylgruppe stehen, wobei aber vorzugsweise mindestens drei, insbesondere mindestens 4 der Reste R1 bis R5 für ein H-Atom stehen.

Bevorzugte Pyrazole der Formel IV sind solche, in denen R1 für eine C1 bis C10 Alkylgruppe steht und R2, R3, R4 unabhängig voneinander für ein H-Atom oder eine C1 bis C10 Alkylgruppe stehen.

Ganz besonders bevorzugte Pyrazole der Formel IV sind solche, in denen R1 für eine C1 bis C4 Alkylgruppe und R2, R3 und R4 jeweils für ein H-Atom stehen.

Ganz besonders bevorzugt handelt es sich bei der Ausgangsverbindung um ein Imidazol der Formel II.

Die Ausgangsverbindung wird mit einem Alkylierungsmittel umgesetzt. Dabei wird vorzugsweise eine C1 bis C10 Alkylgruppe, besonders bevorzugt eine C1 bis C4 Alkylgruppe, insbesondere eine Methylgruppe oder eine Ethylgruppe auf ein Stickstoffatom der Ausgangsverbindung übertragen.

Dazu kommen die üblichen Alkylierungsmittel in Betracht; genannt seien insbesondere Alkylhalogenide, Tri- oder Dialkylphosphate, Mono- oder Dialkylsulfate, Mono- oder Dialkylcarbonate oder einen Alkylester der Alkylsulfonsäure. Bevorzugt sind Alkylhalogenide, Trialkylphosphate, Dialkylsulfate, Dialkylcarbonate oder Alkylester der Alkylsulfonsäure.

Ein besonders geeignetes Alkylierungsmittel ist insbesondere ein Dialkylsulfat der Formel V 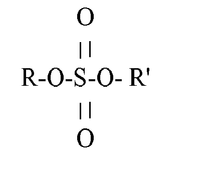 oder ein Dialkylcarbonat der Formel VI 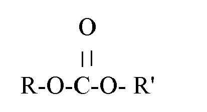 wobei der Rest R für eine Alkylgruppe und R' für ein H-Atom oder eine Alkylgruppe steht.

Der Rest R in Formeln V und VI steht insbesondere eine C1 bis C20-Alkylgruppe. Besonders bevorzugt steht R für eine C1 bis C10 Alkylgruppe, insbesondere eine C1 bis C4 Alkylgruppe.

Der Rest R' in Formeln V und VI steht insbesondere eine C1 bis C20-Alkylgruppe oder ein H-Atom. Besonders bevorzugt steht R' für eine C1 bis C10 Alkylgruppe, insbesondere eine C1 bis C4 Alkylgruppe. Insbesondere haben R und R' die gleiche Bedeutung.

Als Alkylierungsmittel der Formel VI sei insbesondere Dimethylcarbonat genannt.

Ganz besonders bevorzugt sind die Dialkylsulfate der Formel V, besonders bevorzugt Di-C1-C10-alkylsulfate, wie Dimethylsulfat und insbesondere Diethylsulfat.

Die Alkylierung wird in Gegenwart eines Metallsalzes vorgenommen. Dieses Metallsalz ist vorzugsweise keine ionische Flüssigkeit und hat dementsprechend einen Schmelzpunkt größer 100°C (bei Normaldruck, 1 bar).

Vorzugsweise handelt es sich um ein Metallsalz eines Metalls der I, II oder III Hauptgruppe des Periodensystems. Besonders bevorzugt sind Alkali- oder Erdalkalisalze, z.B. Natrium- oder Kaliumsalze.

Das Anion des Metallsalzes kann beliebig sein und bestimmt sich letztlich durch die beabsichtigte Verwendung. Für Verwendungen der erhaltenen Lösungen als Antistatika sind z. B. Anionen ausgewählt aus

Bis(perfluoralkylsulfonyl)amid, Bis(perfluoralkylsulfonyl)imid, wie Bis(trifluormethylsulfonyl)imid, Alkyltosylaten, Aryltosylaten, Perfluoralkyltosylaten, Nitrat, Sulfat, Hydrogensulfat, Alkysulfaten, Arylsulfaten, Polyethersulfaten, Sulfonat, Alkylsulfonaten, Arylsulfonaten, perfluorierte Alkyl- und Arylsulfonaten, Alkylcarboxylaten, Arylcarboxylaten, Perfluoralkylcarboxylaten, Perchlorat, Tetrachloroaluminat, Saccharinat, Thiocyanat, Isothiocyanat, Dicyanamid, Tetraphenylborat, Tetrakis(pentafluorphenyl)borat, Tetrafluoroborat, Hexafluorophosphat, Phosphat, Polyetherphosphate, Methide (Verbindungen mit dreibindigem Kohlenstoff, negativer Ladung am Kohlenstoff), z. B. Tricyanomethid.

### geeignet.

Besonders bevorzugte Metallsalze sind die Lithium-, Natrium- oder Kaliumsalze der vorstehend aufgeführten Anionen, insbesondere von Dicyanamid, Tetrafluoroborat, Hexafluorophosphat, Bis(trifluormethylsulfonyl)imid und Perchlorat.

Ganz besonders bevorzugt sind Natriumdicyanamid (NaN(CN)₂), Natriumtetrafluoroborat (NaBF₄) Lithiumhexafluorophosphat (LiPF₆), Lithiumbis(trifluormethylsulfonyl)imid (kurz LiTFSI), Natriumperchlorat (NaClO₄) oder Kaliumperchlorat (KClO₄).

Zur Durchführung des erfindungsgemäßen Verfahrens wird die Ausgangsverbindung mit dem Alkylierungsmittel in Gegenwart des Metallsalzes umgesetzt.

Die Ausgangsverbindung und das Alkylierungsmittel werden vorzugsweise in einem molaren Verhältnis von 1,5 : 1 bis 1 : 1,5, besonders bevorzugt in einem molaren Verhältnis von 1,2 : 1 bis 1 : 1,2 eingesetzt. Ganz besonders bevorzugt ist ein möglichst equimolares Verhältnis. Das Metallsalz wird in einer Menge eingesetzt, die der später gewünschten Konzentration des Metallsalzes in der ionischen Flüssigkeit entspricht (siehe unten).

Bei der Umsetzung kann auch ein Lösemittel mit verwendet werden. Es ist aber ein Vorteil des erfindungsgemäßen Verfahrens, dass die Mitverwendung eines Lösemittels nicht notwendig, ist um homogene Lösungen herzustellen. Vorzugsweise wird daher das Alkylierungsmittel zur flüssigen Ausgangsverbindung ohne Mitverwendung eines Lösemittels gegeben.

Die Reaktionstemperatur beträgt vorzugsweise 0 bis 200°C, insbesondere 20 bis 150 °C. Die Umsetzung erfolgt vorzugsweise bei Normaldruck, sie kann aber auch unter Druck, z. B. bei Drucken von bis zu 10 bar durchgeführt werden.

Die Umsetzung kann kontinuierlich, semikontinuierlich oder batchweise erfolgen. Insbesondere kann die Ausgangsverbindung zusammen mit dem Metallsalz vorgelegt und das Alkylierungsmittel kontinuierlich zugegeben werden.

Die Umsetzung wird beendet, sobald der gewünschte Umsatz, im Allgemeinen ein Umsatz von mindestens 95 %, vorzugsweise mindestens 98 %, bezogen auf die Ausgangsverbindung erreicht ist.

Erhalten wird eine homogene Lösung des Metallsalzes in der hergestellten ionischen Flüssigkeit.

Die hergestellte Lösung enthält vorzugsweise 1 bis 25 Gew. Teile Metallsalz auf 100 Gew. Teile ionische Flüssigkeit. Es ist ein Vorteil des vorliegenden Verfahrens, dass leicht homogene Lösungen mit einem Gehalt von mindestens 9 Gew. teilen Metallsalz herstellbar sind; Lösungen mit 9 bis 25, insbesondere 9 bis 15 Gew. teilen Metallsalz auf 100 Gew. teile ionische Flüssigkeit sind daher bevorzugt.

Bei den hergestellten ionischen Flüssigkeiten handelt es sich entsprechend der Ausgangsverbindungen gemäß Formeln I bis IV um ionische Flüssigkeiten mit einem Kation ausgewählt aus:
einem quaternären Ammoniumion der Formel VII 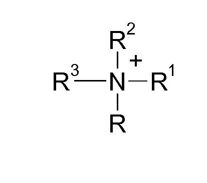
einem Imidazoliumion der Formel VIII 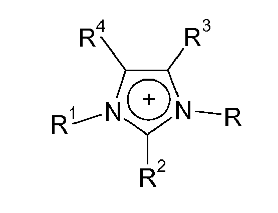 einem Pyridiniumion Formel IX 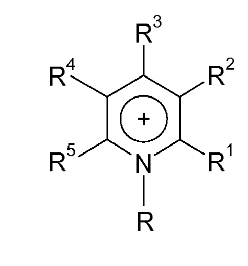
und einem Pyrazoliumion Formel X.

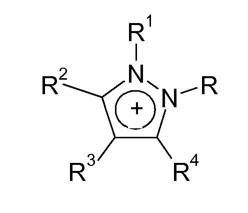

Die Reste R1 bis R5 haben die obige Bedeutung. Der Rest R wurde bei der Alkylierung vom Alkylierungsmittel auf die Ausgangsverbindung übertragen. Der verbleibende Rest des Alkylierungsmittels bildet das Anion. Bei dem Anion handelt es sich dann entsprechend der obigen Alkylierungsmittel um ein Halogenid, Dialkylphosphat, Alkylsulfat, Alkcarbonat oder ein Alkylsulfonat, insbesondere Methansulfonat.

Bei dem zugehörigen Anion handelt es sich besonders bevorzugt um ein Alkylsulfat der Formel XI 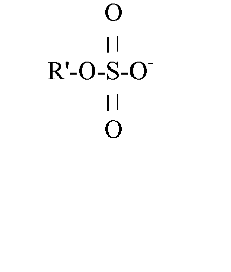 oder ein Alkylcarbonat der Formel XII 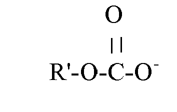 wobei der Rest R' die obige Bedeutung hat.

Ganz besonders bevorzugt werden nach dem erfindungsgemäßen Verfahren Imidazolium-alkylsulfate hergestellt, insbesondere wird 1-Ethyl-3-methylimidazolium-ethylsulfat (EMIM-ethylsufat) hergestellt.

Die erhaltenen Lösungen der Metallsalze in den vorstehenden ionischen Flüssigkeiten sind homogen und stabil, d.h. das Metallsalz fällt auch nach langer Lagerungszeit nicht aus.

Sie eignen sich gut für technische Verwendungen, insbesondere z.B. zur Verwendung als Antistatika. Die Lösung kann daher zunächst gemäß dem erfindungsgemäßen Verfahren hergestellt und anschließend zur antistatischen Ausrüstung von Kunststoff-formteilen, Beschichtungsmitteln und Klebstoffen verwendet werden.

Beispiele:
34,6 g 1-Methylimidazol und 15,53 g Natriumdicyanamid werden bei 50°C vorgelegt. Bei 50 °C werden über einen Zeitraum von 3,5 h 64,6 g Diethylsulfat zugetropft. Anschließend wird die Reaktionsmischung für 24 h bei 50 °C gerührt. Es bleibt eine klare Flüssigkeit mit einem Rest 1-Methylimidazolgehalt von 0,4 %
34,6 g 1-Methylimidazol und 15,53 g Natriumtetrafluoroborat werden bei 50°C vorgelegt. Bei 50 °C werden über einen Zeitraum von 3,5 h 64,6 g Diethylsulfat zugetropft. Anschließend wird die Reaktionsmischung für 24 h bei 50 °C gerührt. Es bleibt eine klare Flüssigkeit mit einem Rest 1-Methylimidazolgehalt von 0,2 %

Die Flüssigkeiten sind stabil, eine Entmischung konnte auch über einen Beobachtungszeitraum von einigen Monaten nicht beobachtet werden.

## Patentansprüche

1. Verfahren zur Herstellung einer Lösung eines Metallsalzes in einer ionischen Flüssigkeit, **dadurch gekennzeichnet, dass**
- die ionische Flüssigkeit durch Alkylierung einer Ausgangsverbindung, welche mindestens ein Stickstoffatom enthält, hergestellt wird und die Alkylierung in Gegenwart des Metallsalzes erfolgt.

2. Verfahren gemäß Anspruch 1, **dadurch gekennzeichnet, dass** es sich bei der Ausgangsverbindung um ein tertiäres Amin mit drei organischen Substituenten oder eine Verbindung mit einem heterocyclischen Ringsystem, welches 1 bis 3 Stickstoffatome enthält, handelt.

3. Verfahren gemäß Anspruch 1 oder 2, **dadurch gekennzeichnet, dass** es sich bei der Ausgangsverbindung um ein tertiäres Amin der Formel I 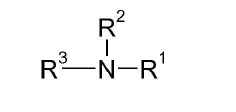 ein Imidazol der Formel II 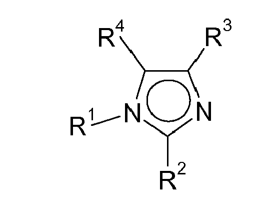 ein Pyridin der allgemeinen Formel III 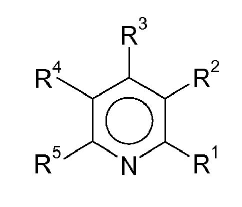 oder ein Pyrazol der allgemeinen Formel IV 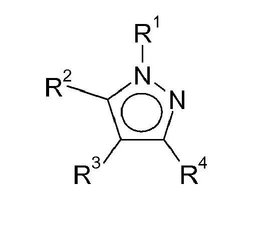 handelt, wobei die Reste R1 bis R5 unabhängig voneinander für ein Wasserstoffatom oder eine organische Gruppe mit 1 bis 20 C-Atomen stehen.

4. Verfahren gemäß einem der Ansprüche 1 bis 3, **dadurch gekennzeichnet, dass** es sich um ein Imidazol der Formel II handelt.

5. Verfahren gemäß einem der Ansprüche 1 bis 4, **dadurch gekennzeichnet, dass** es sich bei dem Alkylierungsmittel um ein Alkylhalogenid, Tri- oder Dialkylphosphat, Mono- oder Dialkylsulfat, Mono- oder Dialkylcarbonat oder einen Alkylester der Alkylsulfonsäure handelt.

6. Verfahren gemäß einem der Ansprüche 1 bis 5, **dadurch gekennzeichnet, dass**
es sich bei dem Alkylierungsmittel um ein Dialkylsulfat der Formel V 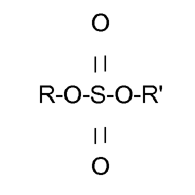 oder ein Dialkylcarbonat der Formel VI 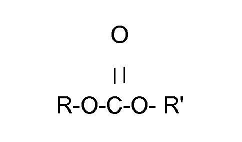 handelt, wobei der Rest R für eine Alkylgruppe und R' für ein H-Atom oder eine Alkylgruppe steht.

7. Verfahren gemäß einem der Ansprüche 1 bis 6, **dadurch gekennzeichnet, dass** es sich bei dem Alkylierungsmittel um ein Di-C1-C10-alkylsulfat handelt.

8. Verfahren gemäß einem der Ansprüche 1 bis 7, **dadurch gekennzeichnet, dass** es sich bei dem Metallsalz um ein Salz eines Metalls der I, II oder III Hauptgruppe des Periodensystems handelt.

9. Verfahren gemäß einem der Ansprüche 1 bis 8, **dadurch gekennzeichnet, dass** es sich um ein Alkali- oder Alkalimetallsalz eines Anions ausgewählt aus
Bis(perfluoralkylsulfonyl)amid, Bis(perfluoralkylsulfonyl)imid, wie Bis(trifluormethylsulfonyl)imid, Alkyltosylaten, Aryltosylaten, Perfluoralkyltosylaten, Nitrat, Sulfat, Hydrogensulfat, Alkysulfaten, Arylsulfaten, Polyethersulfaten, Sulfonat, Alkylsulfonaten, Arylsulfonaten, perfluorierte Alkyl- und Arylsulfonaten, Alkylcarboxylaten, Arylcarboxylaten, Perfluoralkylcarboxylaten, Perchlorat, Tetrachloroaluminat, Saccharinat, Thiocyanat, Isothiocyanat, Dicyanamid, Tetraphenylborat, Tetrakis(pentafluorphenyl)borat, Tetrafluoroborat, Hexafluorophosphat, Phosphat, Polyetherphosphate und Methide, z. B. Tricyanomethid handelt.

10. Verfahren gemäß einem der Ansprüche 1 bis 9, **dadurch gekennzeichnet, dass** es sich bei dem Metallsalz um Natriumdicyanamid (NaN(CN)₂), Natriumtetrafluoroborat (NaBF₄) Lithiumhexafluorophosphat (LiPF₆), Lithium-bis(trifluormethylsulfonyl)imid (kurz LiTFSI), Natriumperchlorat (NaClO₄) oder Kaliumperchlorat (KClO₄) handelt.

11. Verfahren gemäß einem der Ansprüche 1 bis 9, **dadurch gekennzeichnet, dass** die hergestellte Lösung 1 bis 25 Gew. Teile Metallsalz auf 100 Gew. Teile ionische Flüssigkeit enthält.

12. Verfahren zur Verwendung von Lösungen von Metallsalzen in ionischen Flüssigkeiten als Antistatika, **dadurch gekennzeichnet, dass** zunächst eine Lösung gemäß einem der Ansprüche 1 bis 11 hergestellt wird und diese Lösung anschließend zur antistatischen Ausrüstung von Kunststoff-formteilen, Beschichtungsmitteln und Klebstoffen verwendet wird.
